# EUROPEAN PATENT SPECIFICATION

(11) **EP 2 566 421 B1**
(45) Date of publication and mention of the grant of the patent: **19.08.2015**
(21) Application number: 11723672.9
(22) Date of filing: 02.05.2011
(51) Int. Cl.: A61F 5/445

(54) **OSTOMY PORT GAS RELEASE MECHANISM**
OSTOMIEANSCHLUSS MIT ENTLÜFTUNGSVORRICHTUNG
MÉCANISME DE LIBÉRATION DE GAZ POUR ORIFICE DE STOMIE

(30) Priority: 10.01.2011 US 431084 P; 14.07.2010 WO PCT/IL2010/000565; 02.05.2010 US 330359 P
(43) Date of publication of application: 13.03.2013
(73) Proprietor: B. Braun Medical SAS, 92100 Boulogne Billancourt (FR)
(72) Inventor: HANUKA, David, 30095 Ramat-Yishai (IL); OR, Meir, 20196 Doar-Na Misgav (IL)
(74) Representative: Mahler, Peter
(86) International application number: PCT/IB2011/051932
(87) International publication number: WO 2011/138727

(56) References cited:
- WO-A1-01/49224
- US-A- 5 125 916

## Description

### FIELD AND BACKGROUND OF THE INVENTION

The present invention, in some embodiments thereof, relates to ostomy ports and, more particularly, but not exclusively, to a gas release mechanism for an ostomy port.

Controlling gas (flatulation) releasing in an ostomy port is generally desirable for preventing possible risk and/or discomfort to a user of the port resulting from accumulation of intestinal flatus. Embarrassment and/or discomfort may additionally result from unintended releasing of flatus in an undesirable manner to the port's user. Some methods used in the art attempt to solve the problem by including mechanisms in colostomy bags which are attached to the ostomy port, while others include gas release mechanisms in the ports.

U.S. Patent Application Publication No. 2008/0275410 relates to "a colostomy bag with a vent and a method for venting gas collected in the colostomy bag. A dual vent and cap assembly attached to a colostomy bag vents gas trapped in the bag either continuously or as periodically desired by a user. A method for venting gas collected in the colostomy bag provides that replacement and/or cleaning of the colostomy bag is reduced. Also, a method of using a disposable sleeve in combination with a clip may be used to hygienically clean the colostomy bag."

U.S. Patent No. 6,033,390 relates to "a continent ostomy port device has a face plate defining an aperture alignable with the opening of a stoma in the user's body and a closure adjacent to the aperture is adapted to permit covering and uncoveing of the aperture in the face plate. A catheter extends from one side of the face plate proximally, and one end of the catheter is disposed within the ostomy site when the port device is in use. The catheter has continuous exterior and interior side walls, the latter defining a major lumen and is sized and shaped for non-surgical insertion through a stoma to a sufficient distance that the presence of the catheter within the stoma provides a barrier which reduces the incidence of prolapse, without the use of extraneous, externally applied materials or additional surgery. A removable cartridge fits snugly and slideably within the major lumen of the catheter of the device so as to prevent inadvertent escape of body waste material from the stoma when the cartridge is in place, without use of an ostomy bag, and to clean the interior side wall of the catheter as the cartridge is pressed into the major lumen. An anti-reflux valve is activated to prevent escape of body waste and deactivated for passage of fluid. Retaining structure is connected to the catheter, and is non-surgically, snugly fittable into the stoma, to cause the port device to be self-retaining in a normal use position within a stoma, without surgery or fixation materials."

U.S Patent 5,125,916 to Panebianco et al., is considered to represent the closest prior art for independent claim 1 and describes an ostomy appliance for selectively sealing a stoma. The appliance includes a central elongated relatively rigid tube having inner and outer end portions. A cap is support to the outer end portion of the tube and is adapted to engage the users skin when the tube is inserted in a stoma. A flexible extendable and collapsible bellows is mounted on the inner end of the tube for sealing the inside of the stoma when the appliance is inserted therein. A flexible rod insertable through the cap and tube for engagement with the bellows extends the bellows to collapse it for insertion in the stoma.

WO 01/49224 is considered to represent the closest prior art for independent claim 6.

Addidonal background art includes US Patent No. 5,658,267; US Patent No. 4,810,250; US Patent Application Publication No. 2006/0106354 A1; US Patent No. 4,030,500; US Patent No. 4,121,589; US Patent No. 5,045,052; US Patent No. 6,485,476 B1 US Patent Application Publication No. 2008/0275410 A; US Patent No. 4,211,224; US Patent No. 4,338,937; US Patent No. 4,662,890; US Patent No. 5,569,216; US Patent Application Publication No. 2010/0174253; and US Patent No.4,516,974.

### SUMMARY OF THE INVENTION

There is provided in accordance with an exemplary embodiment of the invention a method of controlled gas release from an ostomy port, comprising:
(a) determining that there is a need to release gas from a body of a patient;
(b) temporarily opening a pathway from said body to an outside thereof.

Optionally, said determining comprises determining by an automatic mechanism. Optionally or alternatively, said determining comprises determining by a human. Optionally or alternatively, said determining comprises determining in response to a sensor.

In an exemplary embodiment of the invention, said opening comprises opening a valve in a dedicated exhaust lumen.

In an exemplary embodiment of the invention, said opening comprises opening a gap between said port and a seal thereof.

In an exemplary embodiment of the invention, said pathway includes a stench negating material therein.

In an exemplary embodiment of the invention, said pathway includes a filter therein.

In an exemplary embodiment of the invention, the method comprises repeating said determining and said releasing at least two times in 24 hours, for at least 5 days in a period of 30 days and not while irrigating through said passageway.

There is provided in accordance with an exemplary embodiment of the invention an ostomy port, comprising:
(a) a blocked channel for waste conduction from inside the body to out of the body;
(b) a selectively open gas pathway communicating with one or both of said channel and said inside of the body and which at least partially does not overlap with said waste conduction channel. Optionally, said blocked channel is manually openable. Optionally or alternatively, said gas pathway includes a gap between said channel and a body of said port. Optionally, the port comprises at least one distortable component which, when distorted, provides said gap.

In an exemplary embodiment of the invention, the port comprises at least one movable element which, when moved, provides said gap. Optionally, said motion comprises motion in a plane perpendicular to an axis of said channel

In an exemplary embodiment of the invention, said gas pathway includes at least one dedicated lumen.

In an exemplary embodiment of the invention, said gas pathway includes a plurality of lumens.

In an exemplary embodiment of the invention, said gas pathway includes a plurality of openings into said channel.

In an exemplary embodiment of the invention, said gas pathway includes a stench treating material.

In an exemplary embodiment of the invention, said gas pathway includes a valve. Optionally, said gas pathway includes an automatically actuated valve.

In an exemplary embodiment of the invention, said gas pathway includes a pressure sensor which generates a signal in response to a pressure level.

There is provided in accordance with an exemplary embodiment of the invention an ostomy port, comprising:
(a) a blocked channel for waste conduction from inside the body to out of the body;
(b) an open gas pathway integral to said port communicating with one or both of said channel and said inside of the body and which at least partially does not overlap with said waste conduction channel. Optionally, said gas pathway is a filtering pathway.

There is provided an example of an ostomy port, comprising:
(a) a channel for waste conduction from inside the body to out of the body;
(b) a waste collection bag at a terminus of said channel; and
(c) a gas release pathway configured for releasing gas from inside of said body or said port and which does not pass through said bag.

There is provided in accordance with an exemplary embodiment of the invention an ostomy component, comprising:
(a) a cap suitable for an ostomoy port; and
(b) a coupling device configured to couple said cap to said port and to provide selective gas release.

In an exemplary embodiment of the invention, said coupling device is adapted to rotate relative to said port and provide a gap for gas release from said port. Optionally or alternatively, said coupling device is adapted to be distorted manually and provide a gap for gas release from said port.

There is provided in accordance with an exemplary embodiment of the invention an ostomy component, comprising:
(a) a cap suitable for an ostomoy port; and
(b) a coupling device configured to couple said cap to said port and to provide filtered gas release. Optionally, the component comprises
a waste collection bag attached or integral to said cap.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1 schematically illustrates an exploded perspective view of an exemplary gas release mechanism including a rotary cover for attachment to a stomal cover of an ostomy port for providing a gas flow path from an interior of the ostomy port to the ambient, according to an embodiment of the present invention;
Figure 2 schematically illustrates a perspective view of an underside of the rotary cover shown in Figure 1, according to an embodiment of the present invention;
Figure 3 schematically illustrates the ostomy port with the rotary cover and the fixation ring attached to stomal insert, according to an embodiment of the present invention;
Figure 4A schematically illustrates a sectional view of the gas release mechanism including the rotary cover in the ostomy port in a closed gas flow path configuration;
Figure 4B schematically illustrates a sectional view of the gas release mechanism including the rotary cover in the ostomy port in an open gas flow path configuration;
Figure 4C illustrates a flow chart of a method of releasing gas using the gas release mechanism including the rotary cover;
Figure 4D schematically illustrates a sectional view of the gas release mechanism including the rotary cover and a filter in the ostomy port in the closed gas flow path configuration;
Figure 4E schematically illustrates a sectional view of the gas release mechanism including the rotary cover and the filter in the ostomy port in the open gas flow path configuration;
Figure 5 schematically illustrates an exemplary gas release mechanism including a threaded cover for attachment to a stomal cover in an ostomy port, according to some embodiments of the present invention;
Figures 6A and 6B show schematically illustrates an exemplary gas release mechanism including a cap and a deformable fixation element for attachment to a stomal cover in an ostomy port, according to some embodiments of the present invention;
Figure 7 schematically illustrates an exemplary gas release mechanism having a ventilation port and a lumen for use with an ostomy port having a stomal cover, according to some embodiments of the present invention;
Figures 8A through 8G schematically illustrate exemplary configurations for the lumen, according to some embodiments of the present invention;
Figure 9 schematically illustrates the gas release mechanism with a filter being inserted into the ventilation port, according to some exemplary embodiments of the present invention;
Figure 10A schematically illustrates an exemplary cap with a waste content disposal bag and including a gas filter for use with an ostomy port, according to some embodiments of the present invention;
Figure 10B schematically illustrates the cap following deployment of the waste content disposal bag, according to some embodiments of the present invention; and
Figure 11 is a flow chart illustrating a method of retrofitting an ostomy port with a gas release mechanism including a rotary cover and a fixation element, according to some embodiments of the present invention.

### DESCRIPTION OF SPECIFIC EMBODIMENTS OF THE INVENTION

The present invention, in some embodiments thereof, relates to ostomy ports and, more particularly, but not exclusively, to a gas release mechanism for an ostomy port. The gas release mechanism, in some embodiments, is controlled by a user for actively releasing gas from the interior of the ostomy port to the ambient. In other embodiments, the mechanism includes ventilation means for passively (not user controlled) releasing the gases to the ambient. Actively or passively releasing the gases in the ostomy port may prevent embarrassing situations to the user wherein large discharges of gases are made at one time and/or may prevent discomfort to the user due to gas pressure buildup.

As used hereinafter, the term "distal" refers to away from the proximal opening and towards an interior of the abdominal cavity while "proximal" refers to away from the abdominal cavity towards the proximal opening of the ostomy port (and away from the body).

As used hereinafter, "ostomy port" is any device that is intended to be inserted into a stoma and to be worn on a user's body, and includes a conduit through which waste matter can pass from an intestinal portion inside an abdominal cavity to an outside of the body. The ostomy port may include a stomal cover worn externally on the abdominal wall.

An aspect of some embodiments of the present invention relates to a gas release mechanism having a fixation element attached to a stomal cover of an ostomy port, and a cover movably coupled to the fixation element. The fixation element and the cover are in fluid communication with a proximal opening of the ostomy port for providing a gas flow path from an interior of the ostomy port to the ambient.

In some exemplary embodiments, a cover is rotationally coupled to the fixation element so that rotation of the cover about its axis in a first direction, for example clockwise, opens the gas flow path while rotation in an opposite second direction (e.g. counterclockwise) closes the gas flow path. Additionally or alternatively, rotation in the first direction open the gas flow path while further rotation in the same direction closes the gas flow path.

A particular advantage of some embodiments of the invention is that when manipulating the port, no force is applied towards the body or away from the body. Rather, force is applied in a plane parallel to the body surface and perpendicular to the axis of the ostomy port, for example, translation or rotation forces.

In some exemplary embodiments, the cover is pivotally coupled to the fixation element for opening a gas flow path on an upper section (in the direction of the head of the user) of the proximal opening while maintaining a lower section of the opening sealed to prevent waste content leakage. Optionally, pivoting the cover in a proximal direction relative to the fixation element opens the gas flow path. Returning the pivoted cover to its original position seals the gas flow path. Additionally or alternatively, pivoting the cover laterally relative to the fixation element (in the same plane as the element) opens the gas flow path.

In some exemplary embodiments, the fixation element is annularly shaped and attached to the stomal cover surrounding the proximal opening of the ostomy port. Alternatively, the fixation element is adapted to allow pivoting of the cover relative to the proximal opening. In some embodiments, the fixation element is attachable to ostomy ports known in the art for attaching the gas release mechanisms described herein to the ports when the ports are manufactured. Additionally or alternatively, the gas release mechanisms described herein are retrofitted onto existing ports.

In some exemplary embodiments, the fixation element includes a rigid material such as, for example, a polymer or other plastic material. Alternatively, the fixation element includes a non-plastic material resistant to contact with waste content discharged through the proximal opening. Optionally, the fixation element is attached to the stomal cover using techniques known in the art such as, for example, over-molding, bonding, welding, or any combination thereof.

In some exemplary embodiments, a fastening mechanism between the cover and the fixation ring allows for clockwise and counter-clockwise rotation of the cover while preventing axial displacement of the cover relative to the fixation element. Alternatively, the fastening mechanism includes axial displacement of the cover relative to the fixation element, for example, as when mechanical threads are used.

The threading may be set such that for complete locking, the cover may be rotated by any number of revolutions, from a fraction of a revolution (e.g. ¼ or 1/8 of a revolution) up to a few revolutions (typically less than 3). Optionally, a depth of the threads may be set between 0.5mm and 2 or 3 mm such that attachment between the cover and the stomal cover withstands a pulling force of between 0.5 and 5 kg, for example 2 kg.

Optionally, the fastening mechanism allows for an increase in contact pressure between the cover and the fixation element when the cover is rotated in the clockwise direction and for a decrease in contact pressure when rotation is in the counter-clockwise direction.

In some exemplary embodiments, the cover is annularly shaped having an opening for sealingly accommodating a cap against the stomal cover. Optionally, rotation of the cover in the clockwise direction presses the cap against the fixation element and/or against the ostomy port and seals the proximal opening so that the gas flow path is closed. Additionally, rotation of the cover in the counter-clockwise direction displaces the cap away from the fixation element and from the proximal opening so that the gas flow path is opened. Optionally, cap displacement from sealed position is gradually increased (decreased) as the cover is rotated in the counter-clockwise (clockwise) direction, allowing the user to create a flow path in which only gas can flow but liquid or solid matter is retained in the ostomy port. The gas flow path size, and thereby the amount of displacement of the cap, is set by the user rotating the cover, and may be on the order of tens of microns and optionally not exceeding 500 microns, for example, 50 microns, 70 microns, 100 microns, 150 microns, 200 microns. Optionally, the displacement is greater in an upper section of the cap relative to a lower section of the cap for allowing the gas flow path to be through the upper section and for maintaining a better seal in the lower section for preventing leakage of waste content.

In some exemplary embodiments, the gas release rate is set by the user by the degree to which the rotary slider is rotated. The release rate may vary according to the degree of stool liquidity and on separation between gaseous waste and liquid or solid waste behind the proximal opening of the ostomy port. Optionally, for relatively loose and/or mixed content (where gaseous and liquid/solid waste is mixed distally to the proximal opening), a low release rate may be used, for example on the order of 1 ml/sec, in order to avoid leakage of fecal waste. Additionally, for relatively firm and/or separated content (where mainly gaseous waste is present distally to the proximal opening), higher release rate may be used, for example up to order of 30-50 ml/sec.

In some exemplary embodiments, a locking mechanism secures the cover to the fixation element when the gas flow path is closed, requiring the user to apply a force overcoming the locking mechanism for opening the gas flow path. Optionally, the cover includes a safety catch to prevent undesired removal of the cap from the cover. Optionally, the cap includes a removable cap cover and a deployable waste collection bag for collecting the waste in the ostomy port. Alternatively, the waste collection bag is separately attached to the cap following cap cover removal, or not at all.

An aspect of some embodiments of the present invention relates to a gas release mechanism having a fixation element with an elastically deformable opening which surrounds the proximal opening on the stomal cover. The fixation element sealingly accommodates a cap in the opening when in a pre-deformed state. Optionally, the cap may be similar to that previously described. The deformable opening allows for a gas flow path between an interior of the ostomy port and the ambient when a user presses on a rim of the fixation element for elastically deforming the opening. The fixation ring may be deformable by a pressing force ranging, for example, from 100 - 1000 grams, for example 200 grams, 350 grams, 500 grams, 650 grams, for enabling convenient activation of the gas release mechanism while preventing deformation due to inadvertent forces. The gas flow path size is set by the user pressing on the fixation element, and may be on the order of tens of microns and not exceeding 200 microns, for example, 50 microns, 70 microns, 100 microns, 130 microns, 150 microns. Optionally, the user presses laterally on opposite sides of the rim for deforming the opening. Additionally or alternatively, a clamp may be fitted around the rim which deforms the opening when pressed by the user. Optionally, a bottom section of the opening (in a direction towards the user's legs) is non-deformable so that the user presses on an upper section of the opening for causing the deformation and opening the gas flow path.

In some exemplary embodiments, the fixation element is annularly shaped. Alternatively, the fixation element may be non-circular and may include a circular opening. Optionally, the fixation element may include an elastomer. Additionally or alternatively, the fixation element includes a rigid material, for example, a polymer or other plastic material. Optionally, the fixation element is attached to the stomal cover using techniques known in the art such as, for example, over-molding, bonding, welding, or any combination thereof. Alternatively, the fixation element is an integral part of the stomal cover.

Another aspect of some embodiments of the present invention relates to a gas release mechanism having a ventilation port in the stomal cover in fluid communication with an interior of the ostomy port through at least one lumen. Optionally, the at least one lumen includes an opening at a distal end inside the ostomy port through which gas enters the lumen and flows to the ventilation port (and thereon released into the ambient). Additionally or alternatively, the at least one lumen includes a plurality of openings for gas flow through the lumen to the ventilation port.

A lumen diameter may be in the range of 0.5-3 mm, for example, 1.5 mm, 2 mm, 2.5 mm. A lumen length may extend along the entire length of the ostomy port. Alternatively, the lumen may extend along a portion of the length of the ostomy port. A size of the openings may be on the order of tens of microns and not exceeding 500 microns, for example, 50 microns, 70 microns, 100 microns, 150 microns, 200 microns. The at least one lumen may include a plurality of lumens. Optionally, the at least one lumen is positioned on an upper section of the ostomy port to substantially prevent waste content from entering through the opening or openings. Additionally or alternatively, the openings are of a size which allows gas flow through the openings into the at least one lumen and substantially prevents waste content flow through the opening. In some embodiments, the ventilation port and the at least one lumen can be used for irrigation purposes, an irrigation fluid applied through the ventilation port into the lumen. Additionally or alternatively, the lumen includes open-celled foam for supporting the walls of the lumen while allowing gas flow through the lumen.

In some exemplary embodiments, the ventilation port includes a valve for allowing a user to control the release of gas flow from the interior of the ostomy port. Optionally, the valve includes a stopcock. Alternatively, the valve includes a Schrader valve or other valve suitable for fitting into the ventilation port and for controlling the release of gas. A pressing force by the user, ranging from 100 - 1000 grams, for example 200 grams, 350 grams, 500 grams, 650 grams, may be used to activate the valve for preventing inadvertent operation. Additionally or alternatively, the valve may be automatically activated by a pressure sensing mechanism sensing an increase in pressure in the ostomy port. For relatively loose and/or mixed content (where gaseous and liquid/solid waste is mixed behind the proximal opening), a low release rate may be used, for example on the order of 1 ml/sec, in order to avoid leakage of fecal waste. For relatively firm and/or separated content (where mainly gaseous waste is present behind the proximal opening), higher release rate may be used, for example up to order of 30-50 ml/sec.

In some exemplary embodiments, the ventilation port includes a housing for accommodating a filter for filtering the gas flowing from the interior of the ostomy port. The filter is replaceable and is optionally replaced each time the collection bag is replaced (or emptied in the case of reusable bags), for example, about 1-3 times a day. Filter replacement may require a higher frequency in case of loose and/or mixed content (e.g. in ileostomy) where the filter might be blocked. Optionally, the filter is a charcoal filter or any filter suitable for deodorizing the passing gas so that it will not be perceived by other persons in proximity to the user.

An aspect of some embodiments of the present invention relates to a cap insertable into the proximal opening of the ostomy port and having a gas filter through which gas may flow to the ambient. Optionally, the cap includes a waste collection bag. The filter is located on the cap such that it is exposed to the ambient at all times while the cap in inserted in the proximal opening. Optionally, the filter is exposed to the ambient for providing a gas flow path when the waste content bag is deployed. Alternatively, the filter is on the waste collection bag.

In some exemplary embodiments, a pressure sensor (not shown) is assembled in an interior of the ostomy port, for example on an internal wall of the ostomy port, and a control unit is assembled at a portion of the ostomy port externally to the user's body, for example on the stomal cover. The control unit receives pressure signals from said pressure sensor, and is programmed with a logic algorithm for selectively opening a gas release valve upon fulfillment of predetermined conditions, for example any of the following conditions:
a. Internal pressure is greater than60 mmHg for more than 1 min;
b. Internal pressure is greater than 100 mmHg for more than 10 sec;
c. Internal pressure is greater than 150 mmHg, immediate release.

Optionally, the ostomy port is equipped with an indication mechanism, for example visual, audible or vibrational alarm, or a transmitter or wireless signals (e.g., bluetooth) to notify the user of an activation of the gas release valve.

Additionally or alternatively, the control unit notifies the user on a need to release gas without automatically activating the gas release valve, or with a delay, such as, for example, 1-5 minutes. Optionally, the gas release valve can be closed either manually by the user or automatically by said control unit when the internal pressure decreases, for example, to no greater than 30 mmHg.

In some exemplary embodiments, a pressure sensor and a control unit as those described above control the opening of a gas release valve and/or deploying of a disposable collection bag, according to a predetermined logic, for example:
a. As (e.g., if/when) internal pressure is greater than 60 mmHg for more than 1 min, open the gas release valve;
b. As internal pressure is greater than 60 mmHg for more than 2 min, deploy the disposable collection bag;
c. As internal pressure is greater than 100 mmHg for more than 10 sec, open the gas release valve;
d. As internal pressure is greater than 100 mmHg for more than 30 sec, deploy the disposable collection bag;
e. As internal pressure is greater than 150 mmHg, , open the gas release valve immediately;
f. As internal pressure is greater than 150 mmHg, deploy the disposable collection bag immediately;

Optionally, the pressure sensor includes a mechanical or electro-mechanical control. Additionally or alternative, the pressure sensor may include an override mechanism for allowing the user to decide to open the gas release valve when alerted that the internal pressure has exceeded the predetermined levels. Optionally, the gas release valve can be closed either manually by the user or automatically by a controller when the internal pressure decreases, for example, to no greater than 30 mmHg if the ostomy bag has not been deployed.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

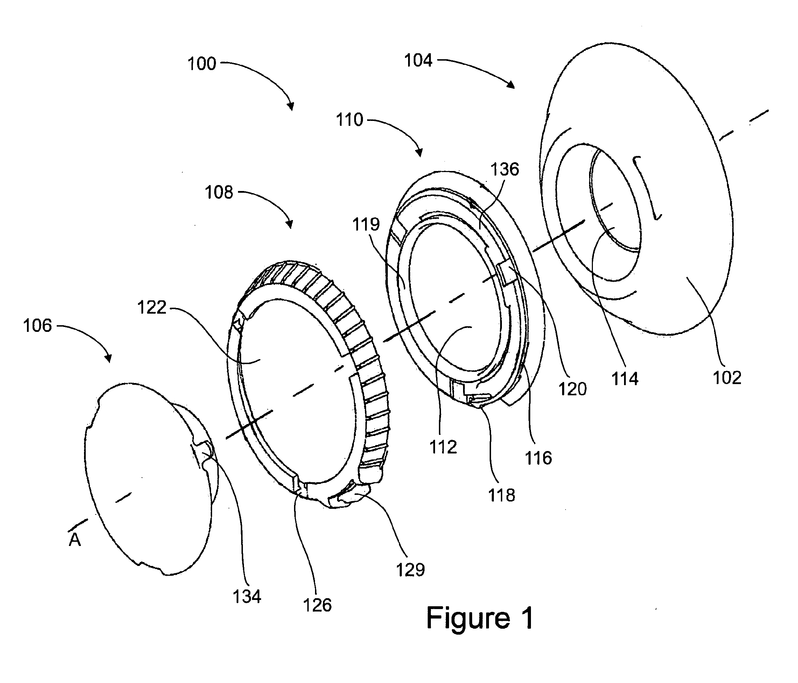

Reference is made to Figure 1 which schematically illustrates an exploded perspective view of an exemplary gas release mechanism **100** for attachment to a stomal cover **102** of an ostomy port **104** for providing a gas flow path from an interior of the ostomy port to the ambient, according to an embodiment of the present invention. Optionally, gas release mechanism **100** includes a cap **106,** a cover **108,** and a fixation element such as, for example, fixation ring **110.**

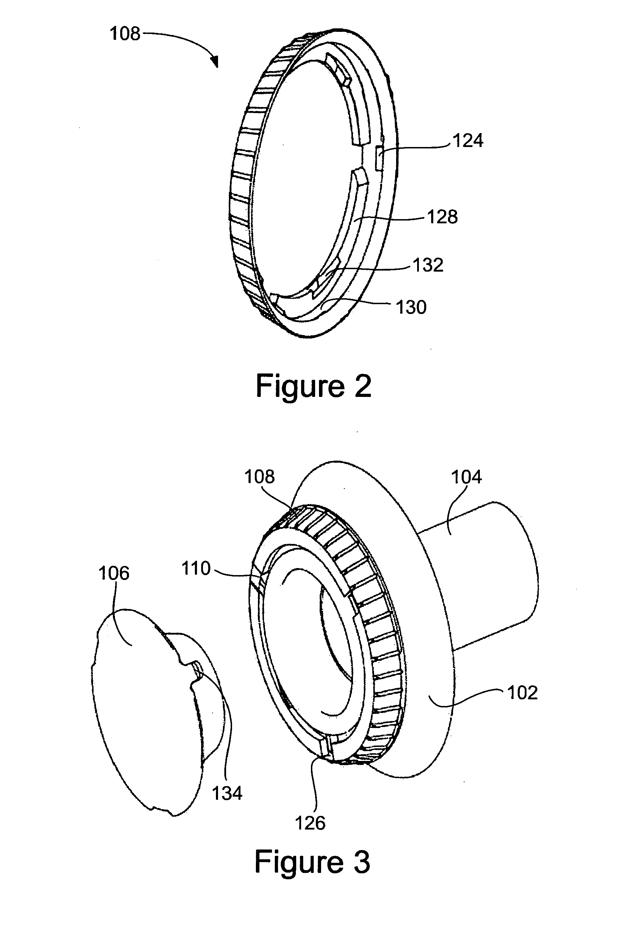

Reference is now also made to Figure 2 which schematically illustrates a perspective view of an underside of cover **108,** and to Figure 3 which schematically illustrates ostomy port **104** with cover **108** and fixation ring **110** attached to stomal cover **102,** according to an embodiment of the present invention.

In some exemplary embodiments, fixation ring **110** is annular shaped and includes an opening **112.** Fixation ring **110** is attached to stomal cover **102** with opening **112** fitting over a proximal opening **114** on the stomal cover, their centers substantially aligned along a central axis A. A recess **116** extends along the whole circumference of fixation element **110.** Alternatively, recess **116** extends along a portion of the circumference of fixation ring **110.** Recess **116** is part of a fastening mechanism which allows clockwise and counterclockwise rotation of cover **108** relative to fixation element **110** and prevents unwanted axial displacement of the cover relative to the fixation element.

A locking mechanism **118** is included for locking cover **108** in position when the gas flow path is closed. Locking mechanism **118** includes a lever which interacts with a recess on cover **108** for providing a warning to a user, for example by a "clicking" noise (sensation) when the cover is locked. Alternatively, locking mechanism **118** locks cover **108** in position when the gas flow path is open.

Further included is a circumferential wall **119** for sealingly securing cap **106** against fixation element **110** when the gas flow path is closed. A plurality of slots **120,** for example three slots, are included in circumferential wall **119** for substantially restricting rotational movement of securing cap **106** relative to fixation element **110** when the gas flow path is closed.

In some exemplary embodiment, cover **108** is annular shaped and includes an opening **122.** Cover **108** is rotably attachable to fixation element **110** with a center of opening **122** substantially aligned with fixation ring **110** along axis A for properly attaching the cover onto the fixation ring.

Included in cover **108** is a plurality of tabs **124,** for example three tabs, forming part of the fastening mechanism coupling cover **108** and fixing element **110.** Tabs **124** fit into recess **116** in fixation ring **110** and slide along the recess, allowing cover **108** to rotate clockwise and counterclockwise. Additionally, tabs **124** are sized to fit into recess **116** for substantially preventing cover **108** displacement in an axial direction.

Further included in cover **108** is a plurality of slots **126,** for example three slots, and a rim **128** extending between the slots along an inside wall **130** of the cover, configured for enabling cap **106** to be inserted into opening **122** and secured to fixation ring **110.** Optionally, rim **128** includes a varying thickness along its whole length for pushing cap **106** in a direction away from proximal opening **114** as cover **108** is rotated for opening the gas flow path. Additionally or alternatively, the varying thickness in rim **128** is for pushing cap **106** towards proximal opening **114** as cover is **108** is rotated for closing the gas flow path. Alternatively, the varying thickness is along a portion of the length of rim **128.** Alternatively, rim **128** includes one or more inclined surfaces **132** for pushing cap **106** in a direction towards proximal opening **114.** Additionally or alternatively, inclined surfaces **132** push cap **106** away from proximal opening. Optionally, rim **128** and/or inclined surfaces **132** may be dimensioned for pushing on cap **106** so that an upper section of the cap is further pushed away from proximal opening **114** relative to a lower section.

Included in cover **108** is safety catch **129** for preventing inadvertent removal of cap **106** from the cover. Safety catch **129** is depressed for allowing cover **108** to be brought to a position where cap **106** may be removed from the cover.

In some exemplary embodiments, cap **106** is adapted to be axially displaced by cover **108** towards proximal opening **114** for closing the gas flow path, or away from the opening for opening the gas flow path, depending on the direction of rotation of the cover. Cap **106** includes a plurality of tabs **134** insertable through slots **126** in cover **108** into slots **120** in fixation ring **110** for sealing proximal opening **114.** Cap **106** may be locked against proximal opening **114** by rotating cover **108** so that tabs **134** are no longer aligned with slots **126** in the cover but are now pressed between rim **128** and slots **120.**

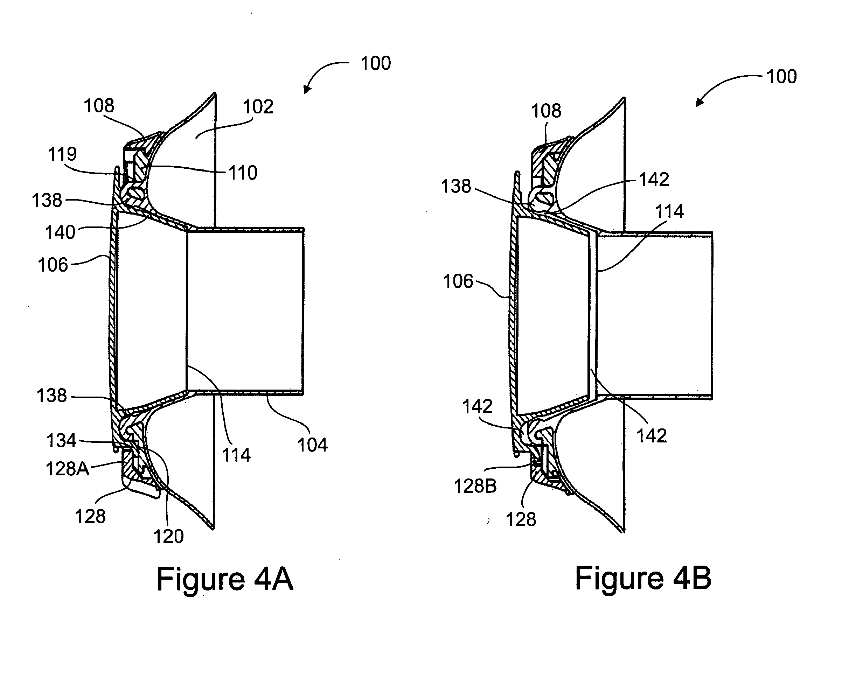

Reference is now also made to Figures 4A and 4B which schematically illustrate sectional views of gas release mechanism **100** in ostomy port **104,** in a closed gas flow path configuration and an open gas flow path configuration, respectively.

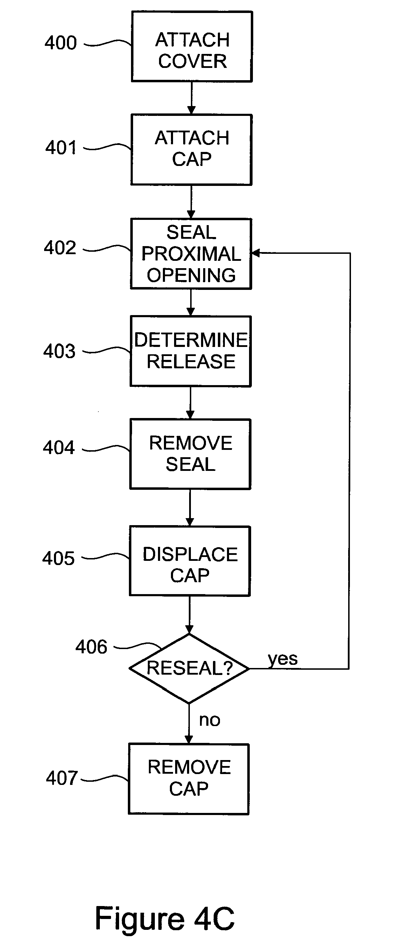

In Figure 4A, cap **106** seals proximal opening **114** for preventing waste contents and gas from escaping from ostomy port **104.** In Figure 4B, cap **106** is displaced away from proximal opening **114** for releasing gas from ostomy port **104** while preventing waste content from leaking out from the ostomy port. To achieve these configurations, reference is now also made to Figure 4C which illustrates a flow chart of a method of releasing gas using gas release mechanism 100.

At **400,** cover **108** is attached to fixation ring **110** and stomal cover **102** in factory. Optionally, stomal cover **102** is part of a new ostomy port **104.** Alternatively, stomal cover **102** and ostomy port **104** are existing and are being retrofitted. Retrofit may be in factory or a retrofit agent.

At **401,** the user introduces cap **106** through opening **122** in cover **108** (the cover was previously attached to fixation ring **110** at **400,** prior to delivery to user), inserting tabs **134** through slots **126** until fitting the tabs in slots **120** in the fixation element.

At **402,** cover **108** is rotated, optionally in a clockwise direction, for securely attaching cap **106** to fixation ring **110** and/or to stomal cover **102** and sealing proximal opening **114.** Tabs **134** are pressed between rim **128** in cover **108** and slots **120** in the fixation element. Optionally, a thicker section **128A** of rim **128** presses on tab **134.** Alternatively, a thicker section of inclined surfaces **132** presses on tab **134.** Optionally, a circumferential seal **138** around proximal opening **114** is pressed between circumferential wall **119** and a side wall **140** of cap **106** and seals proximal opening **114.** In an exemplary embodiment of the invention, seal **138** is compressed to between 70 - 99% of its original thickness, for example 95%. Optionally, circumferential seal **138** is integrally formed as part of stomal cover **102.** In an alternative example, circumferential seal **138** may be a replaceable sealing element, for example an O-ring, fitted around proximal opening **114.**

At **403,** the user determines that gas release is required. Optionally, the user determines release based on experience following a period of time of day or following food consumption. Additionally or alternatively, a pressure sensing mechanism senses an increase in pressure inside the ostomy port and provides a visual and/or audible indication to the user. The indication may be a protruding element which may be seen and/or felt by the user, or an electrical or electromechanical indication such as, for example, a light, a vibration, a sound.

At **404,** the user rotates cover **108,** optionally in a counter-clockwise direction (opposite direction from that in Figure 4A). Rotation of cover **108** causes rim **128** to rotate so that thicker section **128A** no longer presses **tab** 134 against slot **120,** and is replaced by a thinner section **128B** which does not press the tab against the slot.

At **405,** optionally, cap **106** is displaced away from the proximal opening **114** by pressure from the gas inside ostomy port **104,** separating side wall **140** from circumferential seal **138** and opening a gas flow path **142** to the ambient. Optionally, a separation between side wall **120** and circumferential bulge **138** ranges from 10µm to 100µm, 10µm - 70µm, 10µm - 40µm, 10µm - 30µm, 10µm - 20µm. Additionally or alternatively, cap **106** is manually displaced by the user. Optionally, the user may control the displacement of cap **106** by varying an amount of rotation of cover **108.**

At **406,** optionally, following gas release, return to **402** to close gas flow path **142** and seal proximal opening **114.** Alternatively, go to **407** to release cap **106** from ostomy port **104.**

At **407,** rotate cover **102** in the counter-clockwise direction until tabs **134** are aligned with notches **126** and pulls cap **106** from opening **122** in the cover.

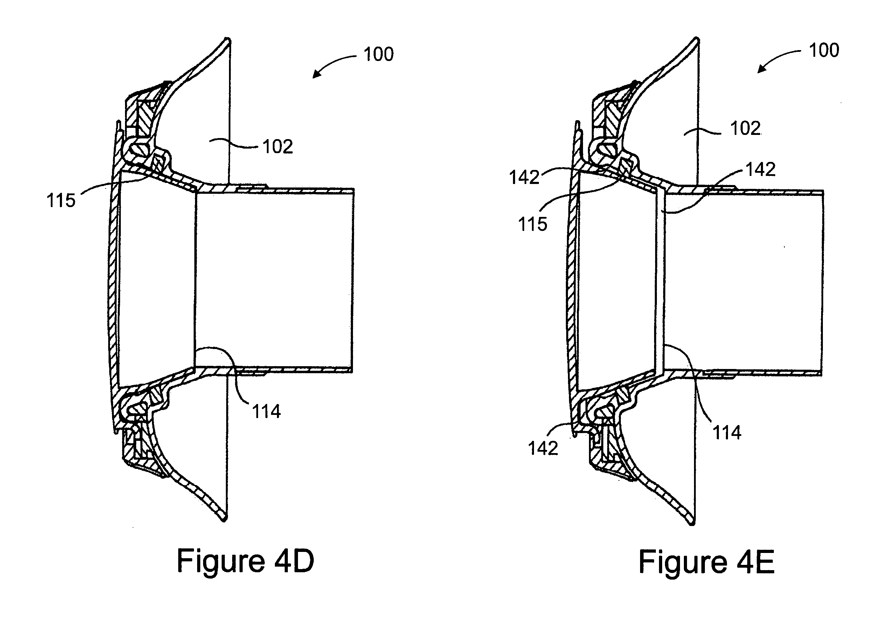

Reference is now also made to Figures 4D and 4E which schematically illustrate sectional views of gas release mechanism **100** including a gas filter **115** in ostomy port **104,** in the closed gas flow path configuration and the open gas flow path configuration, respectively.

In Figure 4D, cap **106** seals proximal opening **114** for preventing waste contents and gas from escaping from ostomy port **104.** In Figure 4E, cap **106** is displaced away from proximal opening **114** for releasing gas from ostomy port **104** while preventing waste content from leaking out from the ostomy port.

In some examples, gas filter **115** is attached to stomal cover **102** so that it lies in gas flow path **142** when gas flow mechanism **100** is in the open gas flow path configuration for filtering the gas flowing therethrough to the ambient. Optionally, the filter is not in the flow and is not used up or contaminated when there is no gas flow.

In an example, filter 115 is annularly shaped. Alternatively, filter 115 encircles only a portion of the circumference of the portion of ostomy port 104 to which it is attached. Optionally, filter 115 is stretchable, such that when cap 106 is pushed in the distal direction into ostomy port 104 (i.e. in the closed gas flow path configuration) filter 115 is stretched radially to accommodate cap 106. Additionally or alternatively, ostomy port 104 is deformable such that when cap 106 is pushed in the distal direction filter 115 is also pushed in the distal direction and ostomy port 104 is deformed to accommodate filter 115 in its altered position. When cap 106 is displaced away from proximal opening 114 (i.e. in the open gas flow path configuration), filter 115 resumes its original shape and position due to its own elasticity. Additionally or alternatively, filter 115 resumes its original shape and position due to the elasticity of the portion of ostomy port 104 to which it is attached.

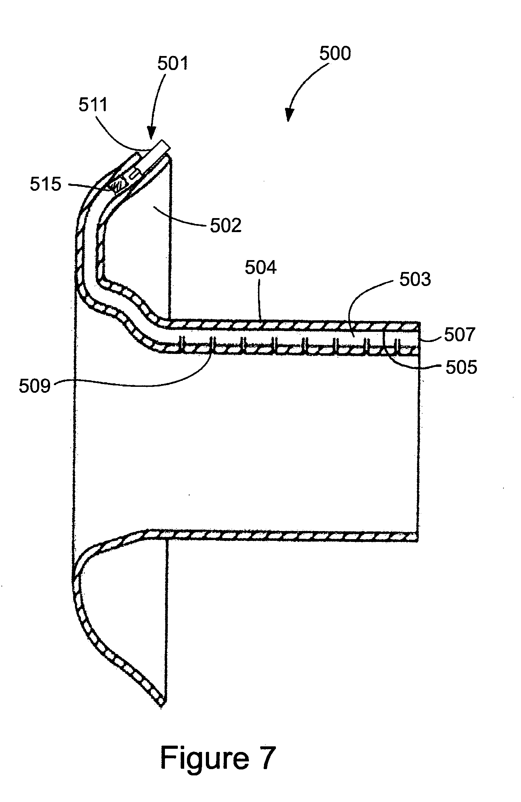

In another example (such as Figure 7, below), a valve may be provided before the filter. In another example, the valve and filter form an element which when moved either block flow along the lumen or allow flow through the filter. In one example, the filter is a cylinder with one half (lengthwise) being a blocking element and fits in a semi-annular portion of a lumen. In another example, the filter is pushed transaxially into the lumen, to displace a blocking of the lumen. In another example, the filter lies in a kink in the lumen, which kink, when straightened allows gas flow through the lumen and the filter.

Optionally, filter **115** includes a charcoal filter element as known in the art. Alternatively, filter **115** may include any other filter element known in the art suitable for insertion into gas flow path **142.** Filter **115** is replaceable and is optionally replaced each time the collection bag is replaced (or emptied in the case of reusable bags), for example, about 1-3 times a day. Filter replacement may require a higher frequency in case of loose and/or mixed content (e.g. in ileostomy) where the filter tends to get blocked.

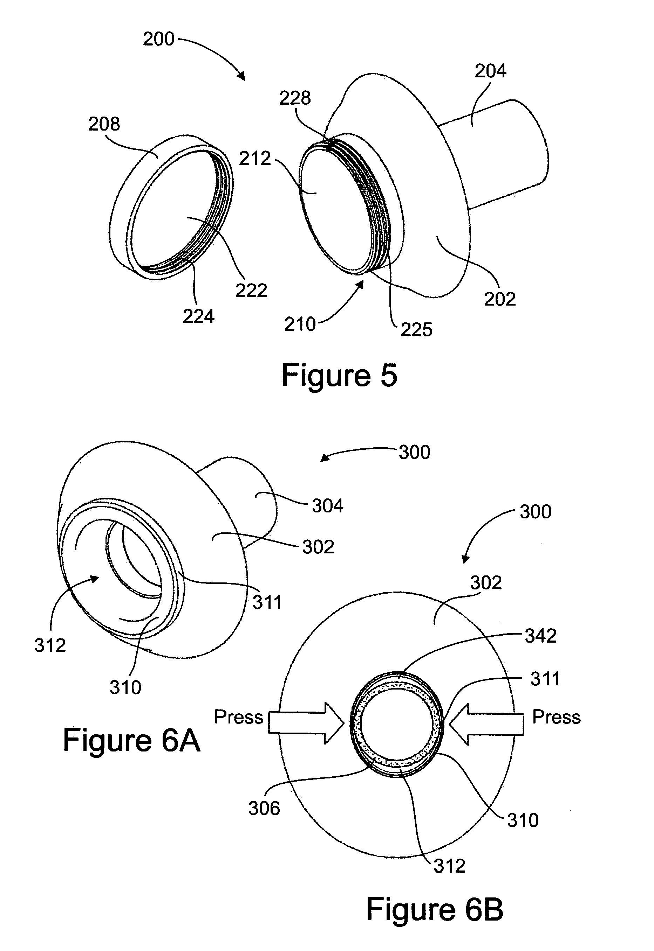

Reference is now made to Figure 5 which schematically illustrates an exemplary gas release mechanism **200** for attachment to a stomal cover **202** in an ostomy port **204,** according to some embodiments of the present invention.

In some exemplary embodiments, gas release mechanism **200** includes a cover **208** having mechanical threads **224** and fixation ring **210** having matching mechanical threads **225.** Optionally, fixation ring **210** is attached to stomal cover **202** using methods known in the art such as, for example, over-molding, welding, bonding, and the like. Alternatively, fixation ring **210** is formed together with, and as part of, stomal cover **202.** Cover **208** is rotably attached to fixation ring **210** by mechanically screwing threads **224** onto threads **225.** Optionally, cover **208** is a closed cover sealing an opening **212** in fixation ring **210** and a proximal opening (not shown) in ostomy port **204.** Optionally, cover **208** and fixation ring **210** are substantially coaxially aligned with a center of the proximal opening. Alternatively, cover **208** includes an opening **222** for inserting a cap (not shown) through opening **212** for sealing ostomy port **104,** the cap being functionally similar to cap **106.** Additionally or alternatively, an ostomy bag (not shown) is attached to opening **222** for allowing waste content to flow out the proximal opening and through opening **222** into the bag.

In some exemplary embodiments, the user seals the proximal opening in ostomy port **204** for preventing escape of waste content and/or gas by screwing cover **208** onto fixation ring **210** and rotating the cover in a clockwise direction until the required sealing is obtained. For releasing gas, the user rotates cover **208** in a counter-clockwise direction until a notch **228** located on an upper section of fixation ring **210** and leading into opening **212** is exposed. Gas may then escape in an upward direction through notch **228** without leakage of waste content. Alternatively, fixation ring **210** does not include a notch and gas may escape through tiny spaces naturally existing between the threading when screwing cover **208** is not sufficiently fastened onto fixation ring **210.** Alternatively, the user seals the proximal opening by rotating cover **208** in a counter-clockwise direction and opens the gas flow path by rotating the cover in the clockwise direction.

Reference is now made to Figure 6 which schematically illustrates an exemplary gas release mechanism **300** for attachment to a stomal cover **302** in an ostomy port **304,** according to some embodiments of the present invention.

In some exemplary embodiments, fixation ring **310** includes an elastically deformable circular opening **312** peripherally bordered by a circumferential rim **311.** Fixation ring **310** is configured to sealingly accommodate a cap **306** for sealing a proximal opening (not shown) in ostomy port **304,** preventing waste content and gas from escaping from ostomy port **304.** Alternatively, the proximal opening is opening **312.** Optionally, cap **306** is functionally similar to cap **106.**

In some exemplary embodiments, for opening a gas flow path from ostomy port **304** to the ambient while cap **306** is inserted in deformable opening **312,** the user laterally presses on circumferential rim **311.** Optionally, lateral pressing may include pressing on circumferential rim **311** on two opposing sections along the circumference. Optionally, the two sections are radially opposed. Laterally pressing on circumferential rim **311** deforms circular opening **312** so that a gas flow path **342** is opened between cap **306** and the circumferential rim. Gas may then escape to the ambient while the waste content remains inside ostomy port **304.** Upon removing the pressing action from circumferential rim **311,** the elastically deformable opening **312** returns to its original shape so that the gas flow path between the circumferential rim and cap **306** is closed and neither waste content nor gas can escape from ostomy port **304.**

Reference is now made to Figure 7 which schematically illustrates an exemplary gas release mechanism **500** including a ventilation port **501** and a lumen **503** in an ostomy port **504,** according to some embodiments of the present invention.

In some exemplary embodiments, ventilation port **501** is located on stomal cover **502** and connects to lumen **503** which extends along a length of an inner wall **505** of ostomy port **504.** Optionally, lumen **503** may include a plurality of lumens extending along inner wall **505.** Optionally, lumen **503** includes a distal opening **507** through which gas from ostomy port **504** enters through the lumen and flows in a direction of ventilation port **501** and out to the ambient. Additionally or alternatively, lumen **503** includes one or more lumen openings **509** along the length of the lumen through which gas enters into the lumen and flows to ventilation port **501.** Optionally, lumen **503** is placed on an upper section of inner wall **505** for allowing rising gases to flow into the lumen while waste content remains on a bottom section of the inner wall. Optionally, ventilation port **501** is placed on an upper section of stomal cover **502** for allowing improved flow of the gases from lumen **503** to the ambient.

In some exemplary embodiments, a valve **511** is connected to ventilation port **501** for allowing the user to control releasing of gas into the ambient. Optionally, valve **511** is a stopcock. Alternatively, valve **511** is a Schrader valve or other type of valve known in the art and suitable for the application.

Referring to valve 511, various valve mechanisms may be used in this or other embodiments of the invention. In an exemplary embodiment of the invention, the valve is a mechanical valve which releases gas when a threshold pressure is passed. In an exemplary embodiment of the invention, the valve is an electronic valve comprising, for example, a pressure sensor, circuitry and an electronically actuated valve component.

In an exemplary embodiment of the invention, the valve generates an alert, for example, vibrating, popping up (e.g., mechanically) or sending a wireless message. Optionally, a transmitter and/or sound or light generator is provided.

In an exemplary embodiment of the invention, the user can select a valve or adjust a valve so as to set the threshold and/or gas release rate.

In an exemplary embodiment of the invention, when used in an airplane or elevator, such a valve can prevent or reduce discomfort caused by changes in ambient air pressure.

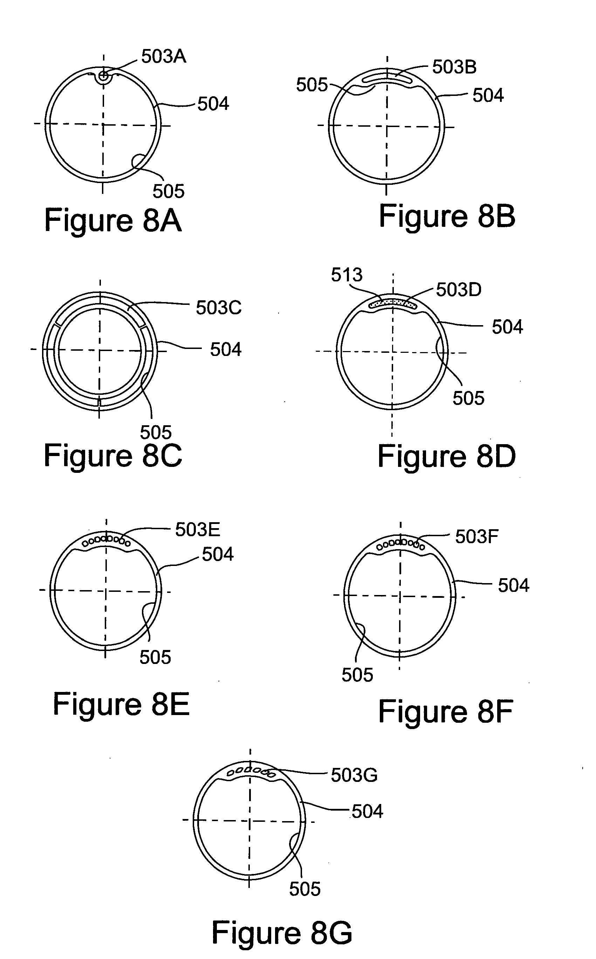

Reference is now also made to Figures 8A through 8G which schematically illustrate exemplary configurations for lumen **503** in Figure 7, according to some embodiments of the present invention. The different configurations may provide for enhanced structural rigidity of the lumen for preventing their possible collapse and blocking of the lumen.

In some exemplary embodiments, lumen **503** has a circular cross-section, as per lumen **503A** in Figure 8A. Alternatively, lumen **503** has an elongated cross-section covering a larger portion of inner wall **505,** as per lumen **503B** in Figure 8B. Alternatively, lumen **503** encircles all of inner wall **505,** as per lumen **503C** in Figure 8C. Additionally or alternatively, lumen **503** is filled with open-cell foam **513,** as per lumen **503D** in Figure 8D. Alternatively, lumen **503** includes a plurality of smaller lumens distributed over a larger portion of inner wall **505,** as per lumen **503E** in Figure 8E, for improving a structural integrity of ostomy port **504.** Optionally, lumen **503E** in Figure 8E can include different shaped smaller lumens, as per lumen **503F** in Figure 8F, and lumen **503G** in Figure 8G.

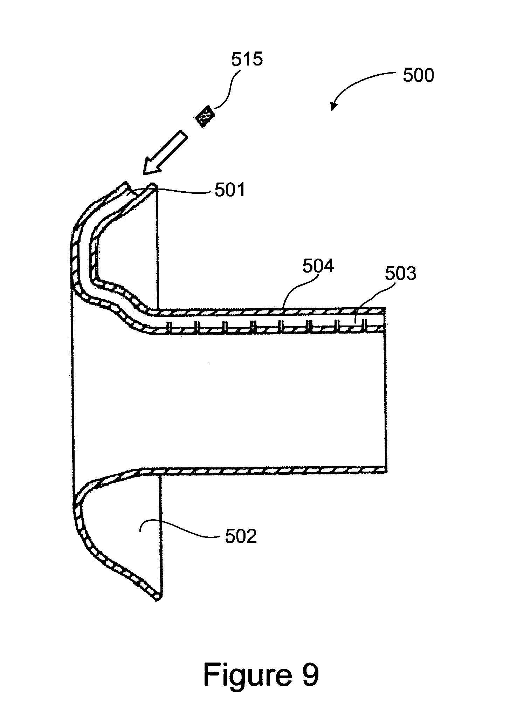

In some exemplary embodiments, replaceable gas filter **515** is insertable into ventilation port **501** for filtering the gas flowing through lumen **503** into the ventilation port prior to release into the ambient. Reference is made to Figure 9 which schematically illustrates gas release mechanism **500** with filter **515** being inserted into ventilation port **501,** according to some exemplary embodiments of the present invention.

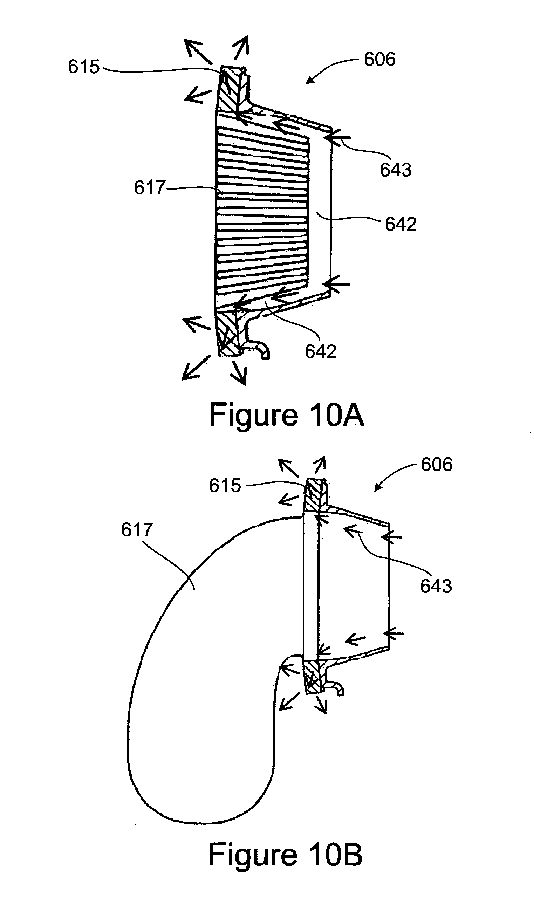

Reference is now made to Figures 10A and 10B which schematically illustrate an exemplary cap **606** with a waste content disposal bag **617** and including a gas filter **615** for use with an ostomy port (not shown), according to some embodiments of the present invention. Optionally, gas filter **615** includes a charcoal filter element. Alternatively, gas filter **615** includes any other filter element known in the art suitable for being used in cap **606.**

While in one embodiment filter 515 and/or filter 615 comprises charcoal, other smell adsorbing materials may be used. Optionally or alternatively, filter 515 is configured to stop particles, for example, being a mesh or a tangle of threads, even if inert. Optionally or alternatively, filter 515 (or 615 or other filters) includes a stench negating function, for example, by smell release and/or by breaking down stench-causing molecules.

In some exemplary embodiments, cap **606** includes waste content disposable bag **617.** Optionally, gas filter **615** is mounted on cap **606** so that it does not interfere with deployment of bag **617.** Optionally, gas filter **615** includes an annular shape and is fitted around margins of cap **606.** Optionally, cap **606** includes a gas flow path **642** around bag **617** directing gas **643** from the ostomy port to gas filter **615** and therefrom to the ambient. Optionally, gas filter **615** continues to filter gas **643** following deployment of bag **617.**

A particular feature of some embodiments of the invention is that gas release can be applied independently of other uses of ostomy port, for example, without risk of becoming dirty (so out in open and not in bathroom), and/or as many times as needed (e.g., in case of gas generation or intestinal discomfort and/or disease.

Gas release can take, for example, between 1 second and five minutes. Optionally, the patient/user moves about to assist in gas exit. Exemplary release times are 1-2 minutes. In an exemplary embodiment of the invention, release is a daily occurrence, for example, being done 1-7 times a day, significantly more often than bag replacement or waste exit and/or at least 10 minutes before or after waste exit. Optionally, an extension tube (not shown), for example, between 3 and 50 cm long, is provided on the exit port to allow a user to aim the exit direction of the gas. Optionally, the tube includes a manual valve at it tip.

As it is a regular occurrence, gas may be periodically released for several days in a row, for example, 5, 10, 20 days or more over long periods of time, such as 1-6 weeks or more. Optionally, the circuitry is set to automatically release gas at certain times (e.g., during sleep) and/or periods and/or in response to motion detection (e.g., acceleration or lack thereof).

In some embodiments of the invention, kits are provided, generally in sterile packaging.

In an exemplary embodiment of the invention, components as described herein are sold in kit form. For example, an ostomy port may be sold with one or more caps and/or one or more filters or valves.

In another example, a retrofit coupling device may be sold, matched to a particular port and/or bag designs.

In another example, a set of replacement filters and/or replacement bags and/or caps may be packaged together, for example, as a package of 5, 10, 20 or smaller, intermediate or larger numbers.

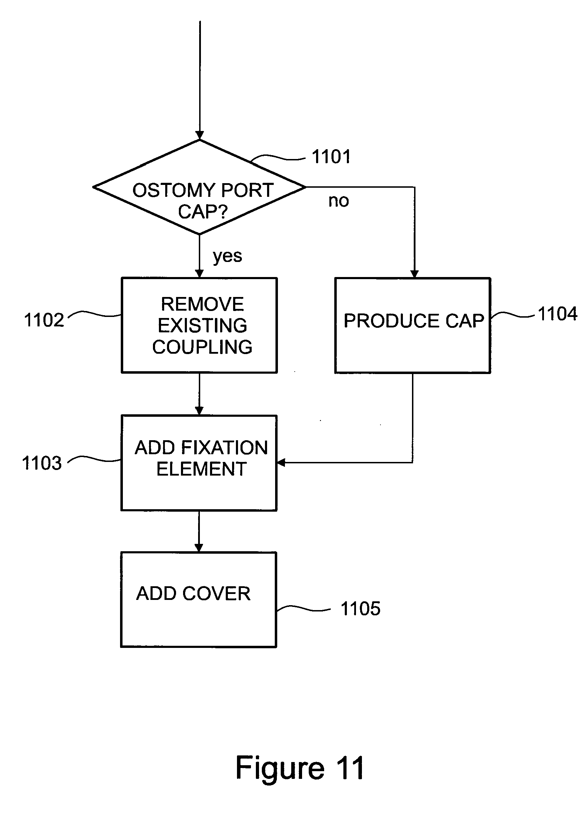

Reference is now made to Figure 11 which is a flow chart illustrating a method of retrofitting an ostomy port with a gas release mechanism including a rotary cover and a fixation element, according to some embodiments of the present invention.

At **1101,** a decision is made by the retrofitter as to whether or not the ostomy port includes a cap used for sealing a proximal opening in the port. Exemplary ports having caps are described in U.S. Patent Nos. 4,121,589; 4,338,937; and 6,033,390. Exemplary ports not having caps are described in U.S. Patent Application Publication NO. 2010/0174253 and U.S. Patents Nos. 4,662,890 and 5,569,216. If the port has a cap, go to **1102.** Otherwise, go to **1104.**

At **1102,** remove any existing coupling mechanism between the ostomy port and the cap.

At **1103,** add in a proximity to the proximal opening of the port a fixation element surrounding this opening. Go to **1105.**

At **1104,** produce a cap capable of sealingly closing the proximal opening of the ostomy port. Go to **1103.**

At **1105,** add a cover adapted to accommodate said cap and to rotate on said fixation element, such that rotation of the cover in one direction creates a sealing contact between the cap and the ostomy port, and rotation of the cover in the other direction loosens said sealing contact, thus creating a gas flow path from an interior of the ostomy port.

The terms "comprises", "comprising", "includes", "including", "having" and their conjugates mean "including but not limited to".

The term "consisting of means "including and limited to".

The term "consisting essentially of" means that the method or structure may include additional steps and/or parts, but only if the steps and/or parts do not materially alter the basic and novel characteristics of the claimed method or structure.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the present invention.

## Claims

1. A method of controlled gas release from an ostomy port (104), comprising:
(a) determining that there is a need to release gas from a body of a patient; and
(b) releasing said gas from said body to an outside thereof;
**characterized by** said gas being released by manipulating a selectively open gas release pathway (142) which at least partially does not overlap with a waste conduction channel in said ostomy port (104).

2. A method according to claim 1, wherein said manipulating said pathway comprises one of opening a valve (511) in a dedicated exhaust lumen (503) and opening a gap between said port (104) and a seal thereof (138).

3. A method according to either of claims 1 and 2, comprising repeating said determining and releasing at least two times in 24 hours, for at least 5 days in a period of 30 days and not while irrigating through said pathway (142).

4. A method according to claim 1 wherein said manipulating the pathway includes adjusting a release rate of said gas to between 1 ml/sec and 50 ml/sec.

5. A method according to claim 1, wherein said pathway (642) includes at least one of a stench negating material (615) and a filter (515) therein.

6. An ostomy port (104), comprising:
(a) a blocked channel for waste conduction from inside the body to out of the body; and
(b) a gas pathway (142) communicating with one or both of said channel and said inside of the body and which at least partially does not overlap with said waste conduction channel;
**characterized in that** said gas pathway (142) is selectively openable.

7. A port (104) according to claim 6, wherein said blocked channel is manually openable.

8. A port (304) according to claim 6, wherein said gas pathway includes a gap between said channel and a body of said port, said port
further comprising one of:
at least one distortable component (311) which, when distorted, provides said gap (342); and
at least one movable element (208) which, when moved, provides said gap.

9. A port (204) according to claim 8, wherein said port comprises at least one movable element (208) which, when moved, provides said gap; and
wherein said motion comprises motion in a plane perpendicular to an axis of said channel.

10. An ostomy port (100) according to claim 6, further comprising a waste collection bag (617) at a terminus of said channel; and wherein said gas pathway (642) does not pass through said bag.

11. An ostomy port (104) according to any of claims 6-10, further comprising:
(a) a cap (106); and
(b) a coupling device (110) configured to couple said cap (106, 108) to said port (104) and to provide selective gas release.

12. An ostomy port (104, 204) according to claim 11, wherein said coupling device (108, 208; 310) is one of adapted to rotate relative to said port and adapted to be distorted manually to provide a gap (342), for gas release from said port (304).

13. A port (104) according to any of claims 6-12, wherein said gas pathway (142) includes a stench treating material (115).

14. A port (504) according to any of claims 6-13, wherein said gas pathway (503) includes a valve (511).

15. A port (504) according to claim 14, wherein said gas pathway (503) includes an automatically actuated valve (511).

16. An ostomy port (104) according to any of claims 6-10, further comprising:
(a) a cap (106, 108); and
(b) a coupling device (110) configured to couple said cap (106, 108) to said port (104) and configured to provide filtered gas release.

17. An ostomy port (104) according to any of claims 11-15, wherein said coupling device (110) is configured to provide filtered gas release.

## Patentansprüche

1. Verfahren zur kontrollierten Entlüftung eines Ostomieanschlusses (104), umfassend:
(a)feststellen, dass ein Bedarf zur Freisetzung von Gas aus dem Körper eines
Patienten vorhanden ist; und
(b)freisetzen des Gases aus dem Körper nach außen;
**dadurch gekennzeichnet, dass** das Gas durch Manipulation einer wahlweise zu öffnenden Entlüftungsleitung (142) freigesetzt wird, die zumindest teilweise nicht mit einem Abfallableitungskanal in dem Ostomieanschluss (104) überlappt.

2. Verfahren nach Anspruch 1, wobei das Manipulieren der Leitung das Öffnen eines Ventils (511) in einem zugehörigen Entlüftungslumen (503) oder das Öffnen einer Öffnung zwischen dem Ostomieanschluss und einer Dichtung davon (108) umfasst.

3. Verfahren nach Anspruch 1 oder 2, welches das wiederholte Feststellen und Entlüften mindestens zweimal in 24 Stunden, für mindestens 5 Tage in einem Zeitraum vom 30 Tagen umfasst, wobei Vorstehendes nicht während der Spülung durch die Entlüftungsleitung (142) geschieht.

4. Verfahren nach Anspruch 1, wobei das Manipulieren der Leitung das Einstellen einer Entlüftungsrate des Gases zwischen 1 ml/s und 50 ml/s beinhaltet.

5. Verfahren nach Anspruch 1, wobei die Leitung (642) zumindest ein geruchsvernichtendes Material (615) oder einen Filter (515) umfasst.

6. Ein Ostomieanschluss (104) umfassend:
(a)einen versperrten Kanal um Abfallstoffe vom Inneren des Körpers nach außerhalb des Körpers zu führen; und
(b)eine Entlüftungsleitung (142), die mit dem Kanal und/oder dem Inneren des Körpers kommuniziert und die zumindest teilweise nicht mit dem Abfallableitungskanal überlappt,
**dadurch gekennzeichnet, dass** die Entlüftungsleitung wahlweise zu öffnen ist.

7. Anschluss (104) nach Anspruch 6, wobei der versperrte Kanal manuell zu öffnen ist.

8. Anschluss (304) nach Anspruch 6, wobei die Entlüftungsleitung eine Öffnung zwischen dem Kanal und einem Körper des Anschlusses aufweist und der Anschluss weiter eines der folgenden Elemente aufweist:
zumindest eine deformierbare Komponente (311), welche im deformierten Zustand die Öffnung (342) erzeugt; und
zumindest ein bewegliches Element (208), welches nach einer Bewegung die Öffnung erzeugt.

9. Anschluss (204) nach Anspruch 8, wobei der Anschluss zumindest ein bewegliches Element (208) umfasst, welches nach einer Bewegung die Öffnung erzeugt; und
wobei diese Bewegung eine Bewegung in einer Ebene senkrecht zu einer Achse des Kanals umfasst.

10. Ostomieanschluss (100) nach Anspruch 6, welcher zusätzlich eine Abfallsammeltasche (617) an einem Ende des Kanals umfasst; wobei die Entlüftungsleitung (642) nicht durch die Tasche führt.

11. Ostomieanschluss nach einem der Ansprüche 6 - 10, weiter umfassend:
(a)eine Kappe (106); und
(b)ein Anschlussstück (110), welches konfiguriert ist, um die Kappe (106, 108) mit dem Anschluss (104) zu verbinden und eine wahlweise Entlüftung zu ermöglichen.

12. Ostomieanschluss (104, 204) nach Anspruch 11, wobei das Anschlussstück (108, 208; 310) derart gestaltet ist, das es relativ zum Anschluss rotieren kann oder manuell deformierbar ist, um eine Öffnung (342) für die Entlüftung des Anschlusses (304) zu erzeugen.

13. Anschluss (104) nach einem der Ansprüche 6 - 12, wobei die Entlüftungsleitung (142) ein geruchsbehandelndes Material (115) aufweist.

14. Anschluss (504) nach einem der Ansprüche 6 -13, wobei die Entlüftungsleitung (503) ein Ventil (511) aufweist.

15. Anschluss (504) nach Anspruch 14, wobei die Entlüftungsleitung (503) ein automatisch betätigtes Ventil (511) aufweist.

16. Ostomieanschluss (104) nach einem der Ansprüche 6 - 10, der zusätzlich umfasst:
(a)eine Kappe (106, 108); und
(b)ein Anschlussstück (110), um die Kappe (106, 108) mit dem Ostomieanschluss (104) zu verbinden und eine gefilterte Entlüftung zu ermöglichen.

17. Ostomieanschluss (104) nach einem der Ansprüche 11 - 15, wobei das Anschlussstück (110) derart konzipiert ist, dass es eine gefilterte Entlüftung ermöglicht.

## Revendications

1. Procédé de libération de gaz contrôlée d'un orifice de stomie (104), comprenant :
(a) détermination qu'il y a un besoin de libérer du gaz du corps d'un patient ;
(b) libération dudit gaz dudit corps vers l'extérieur du corps ;
**caractérisé en ce que** ledit gaz est libéré par la manipulation d'un passage de gaz (142) ouvrable sélectivement qui au moins partiellement n'a pas de chevauchement avec un conduit de matières fécales dans ledit orifice de stomie.

2. Procédé selon la revendication 1, où ladite manipulation dudit passage comprend une des étapes suivantes : ouverture d'une valve (511) dans un lumen d'échappement (503) dédié et ouverture d'une ouverture entre ledit orifice de stomie (104) et un joint de celui-ci (138).

3. Procédé selon la revendication 1 ou 2, comprenant la répétition de ladite détermination et de ladite libération au moins deux fois en 24 heures, pendant au moins 5 jours d'une période de 30 jours et non pendant l'irrigation par ledit passage de gaz (142).

4. Procédé selon la revendication 1, où ladite manipulation du passage comprend l'ajustement d'un taux de libération dudit gaz à entre 1 ml/s et 50 ml/s.

5. Procédé selon la revendication 1, où ledit passage (642) est muni au moins d'un matériau (615) absorbant des odeurs ou d'un filtre (515).

6. Orifice de stomie (104) comprenant :
(a) un conduit bloqué pour le guidage de matières fécales de l'intérieur du corps vers l'extérieur du corps ; et
(b) un passage de gaz (142) en communication avec ledit conduit et/ou ledit intérieur du corps et qui au moins partiellement n'a pas de chevauchement avec ledit conduit de matières fécales ;
**caractérisé en ce que** ledit passage de gaz (142) peut être ouvert sélectivement.

7. Orifice de stomie (104) selon la revendication 6 où ledit conduit bloqué peut être ouvert manuellement.

8. Orifice de stomie (304) selon la revendication 6, où ledit passage de gaz comprend une ouverture entre ledit conduit et un corps dudit orifice, ledit orifice comprenant en outre un des éléments suivants :
au moins une composante déformable (311) qui, quand déformée, fournit ladite ouverture (342) ; et
au moins un élément déplaçable (208) qui, quand déplacé, fournit ladite ouverture.

9. Orifice de stomie (204) selon la revendication 8, où ledit orifice comprend au moins un élément déplaçable (208) qui, quand déplacé, fournit ladite ouverture ; et
où ledit mouvement comprend un mouvement dans un plan perpendiculaire à un axe dudit conduit.

10. Orifice de stomie (100) selon la revendication 6, comprenant en outre une poche (617) pour la collecte des matières fécales à une extrémité dudit conduit ; et où ledit passage de gaz (642) ne passe pas par ladite poche.

11. Orifice de stomie (104) selon l'une quelconque des revendications 6 - 10, comprenant en outre :
(a) un couvercle (106) ; et
(b) un dispositif de couplage (110) apte à coupler ledit couvercle (106, 108) audit orifice (104) et à fournir l'évacuation sélective des gaz.

12. Orifice de stomie (104, 204) selon la revendication 11, où ledit dispositif de couplage (108, 208 ; 310) est apte à tourner relatif audit orifice ou à être déformé manuellement pour fournir une ouverture (342) pour libérer des gaz dudit orifice (304).

13. Orifice de stomie (104) selon l'une quelconque des revendications 6 - 12, où ledit passage de gaz (142) comprend un matériau (115) traitant des odeurs.

14. Orifice de stomie (104) selon l'une quelconque des revendications 6 - 13, où ledit passage de gaz (503) comprend une valve (511).

15. Orifice de stomie (104, 204) selon la revendication 14, où ledit passage de gaz (503) comprend une valve (511) activée automatiquement.

16. Orifice de stomie (104) selon l'une quelconque des revendications 6 - 10, comprenant en outre :
(a)un couvercle (106, 108) ; et
(b)un dispositif de couplage (110) apte à coupler ledit couvercle (106, 108) audit orifice (104) et apte à fournir une libération des gaz filtrée.

17. Orifice de stomie (104) selon l'une quelconque des revendications 11 - 15, où ledit dispositif de couplage est apte à fournir une libération de gaz filtrée.
